(19) Europäisches Patentamt
European Patent Office
Office européen des brevets

(11) Veröffentlichungsnummer: **0 084 833**
**B1**

(12) # EUROPÄISCHE PATENTSCHRIFT

(45) Veröffentlichungstag der Patentschrift: **12.12.84**

(51) Int. Cl.³: **C 07 C 31/08**, C 07 C 31/10, C 07 C 29/32

(21) Anmeldenummer: **83100334.8**

(22) Anmeldetag: **17.01.83**

(54) **Verfahren zur Herstellung von Ethanol und n-Propanol aus Methanol und Synthesegas.**

(30) Priorität: **21.01.82 DE 3201665**

(43) Veröffentlichungstag der Anmeldung: **03.08.83 Patentblatt 83/31**

(45) Bekanntmachung des Hinweises auf die Patenterteilung: **12.12.84 Patentblatt 84/50**

(84) Benannte Vertragsstaaten: **BE DE FR GB IT NL SE**

(56) Entgegenhaltungen:
**EP - A - 0 014 747**
**EP - A - 0 014 747**

**Patent Abstracts of Japan Band 6, Nr. 219, 2. November 1982**

(73) Patentinhaber: **Ruhrchemie Aktiengesellschaft, Bruchstrasse 219, D-4200 Oberhausen 13 (DE)**

(72) Erfinder: **Cornils, Boy, Dr. Dipl.-Chem., Friedrich-Ebert-Strasse 45, D-4220 Dinslaken (DE)**
Erfinder: **Frohning, Carl-Dieter, Dr. Dipl.-Chem., Eisenbruch 1, D-4200 Oberhausen 13 (DE)**
Erfinder: **Bahrmann, Helmut, Dr. Dipl.-Chem., Rohstrasse 48, D-4236 Hamminkeln/Brünen (DE)**
Erfinder: **Lipps, Wolfgang, Dr. Dipl.-Chem., Falkestrasse 76, D-4200 Oberhausen 11 (DE)**

(74) Vertreter: **Reichelt, Karl-Heinz, Dr., m. Br. Ruhrchemie Aktiengesellschaft Abt. PLD Postfach 13 01 60, D-4200 Oberhausen 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft ein Verfahren zur Herstellung von Ethanol und n-Propanol aus Methanol und Synthesegas, d. h. dem Gemisch von Kohlenmonoxid und Wasserstoff in Gegenwart von Kobalt und Ruthenium als Katalysatoren. Diese Reaktion, sie wird als Homologisierung bezeichnet, ermöglicht es, ausgehend von Methanol, durch Einführung einer oder mehrerer $CH_2$-Gruppen höhere homologe Alkohole herzustellen.

Die Homologisierung findet im verstärkten Maße Interesse, da sie einen Weg zur Gewinnung höherer Alkohole eröffnet, der unabhängig von der Verwendung von Erdöl ist. Als Einsatzstoff wird Synthesegas bzw. daraus hergestelltes Methanol benötigt; Synthesegas ist, z. B. aus Kohle oder Erdgas nach verschiedenen, technisch bewährten Prozessen zugänglich.

Es ist bekannt (vgl. z. B. DE-PS 867 849), Methanol mit Wasserstoff und Kohlenmonoxid in Gegenwart eines Kobaltkatalysators bei hohen Temperaturen und Drücken in Ethanol umzuwandeln. Die Reaktion verläuft nach folgender Summengleichung:

$$CH_3OH + CO + 2H_2 \rightarrow C_2H_5OH + H_2O$$

wobei in untergeordnetem Maße höhere Alkohole entsprechend

$$CH_3OH + n(CO + 2H_2) \rightarrow CH_3(CH_2)_nOH + n\ H_2O$$

gebildet werden können.

Während ursprünglich für die Reaktion ausschließlich Kobalt als Katalysator verwendet wurde, gewannen im Laufe der Zeit Mehrkomponenten-Katalysatoren zunehmend an Bedeutung.

In der EP-OS 22 038 ist die Herstellung von Ethanol aus Methanol unter Verwendung von Kobalt als Katalysator, einer organischen und anorganischen Jodverbindung, Ammonium- bzw. Phosphoniumverbindungen sowie einer Rutheniumverbindung als Promotoren beschrieben.

Die gleichzeitige Produktion von Ethanol und Acetaldehyd wird unter Verwendung eines Katalysatorsystems aus einer Kobalt-, einer Jod- und einer zweizähnigen Phosphinkomponente in der EP-OS 10 373 beschrieben. Durch Zugabe eines inerten Lösungsmittels soll die Selektivität zu Ethanol gesteigert werden. Als weitere Katalysatorkomponente soll Ruthenium einsetzbar sein.

Nach der Lehre der US-PS 4 133 966 gewinnt man Ethanol aus Synthesegas und Methanol unter Verwendung eines aus Kobaltacetylacetonat, einer organischen Verbindung eines Elements der Gruppe VA des Periodensystems der Elemente, einer Rutheniumverbindung und einer Jodverbindung bestehenden Katalysators.

In der GB-PS 2 036 739 wird gleichfalls die Verwendung eines Katalysators bestehend aus Kobalt, Ruthenium oder Osmium, Halogen und einer organischen Verbindung eines Elements der Gruppe VA des Periodensystems zur Herstellung von Ethanol aus Methanol und Synthesegas beansprucht.

Kobalt, Ruthenium, Jod und ein tert. Phosphin als Katalysatorkomponente werden auch in der US-PS 4 233 466 zur Herstellung von Ethanol aus Methanol und Synthesegas verwendet mit der Maßgabe, daß das Verhältnis der einzelnen Komponenten innerhalb gewisser Grenzen u einer Stabilisierung und Aktivitätssteigerung des Katalysatorsystems führt.

Ein Katalysatorsystem zur Herstellung von Ethanol aus Methanol bestehend aus einem dimeren Kobalt-tricarbonyl-Ligand-Komplex, dessen Ligand aus einer Verbindung eines Elements der Gruppe VA des Periodensystems der Elemente besteht sowie Jod und Rutheniumacetylacetonat als Promotoren enthält, beschreibt die US-Patentschrift 4 239 924.

Die EP-OS 30 434 beschreibt ein Verfahren zur Homologisierung von Methanol mit Synthesegas in Gegenwart eines inerten Lösungsmittels und einem Katalysatorsystem aus definierten Kobalt-Ruthenium-Komplexen, jodhaltigen Promotoren und tert. Phosphinen oder Phosphiten. An Stelle der definierten Co/Ru-Komplexe sollen auch lösliche Ru-Komplexe zusammen mit $Co_2(CO)_{8-n}(PR_3)_n$-Komplexen einsetzbar sein.

Trotz der vorstehend beschriebenen Maßnahmen reichen die erzielten Ausbeuten der Reaktion für eine technische Anwendung nicht aus. Hierbei ist zu berücksichtigen, daß die Nebenprodukte nicht nur in großer Menge, sondern in Form zahlreicher, unterschiedlicher Einzelverbindungen anfallen. So werden neben den erwünschten Alkoholen z. B. Methan, Ethan, Propan, verschiedene Ether sowie Methylacetat, Ethylacetat, Propylacetat, Acetaldehyd-dimethyl-acetal, Acetyldehyd-methyl-ethylacetal und Acetaldehyd-diethylacetal gebildet. Bei industrieller Nutzung des Prozesses ist daher ein hoher Aufwand erforderlich, um die aus den Nebenprodukten gewinnbaren Wertproduktanteile, z. B. durch Hydrierung, Verseifung und Destillation, zu isolieren.

Eine Verbesserung der Selektivität der Umsetzung durch Zusatz eines Lösungsmittels zu den Reaktionspartnern hat eine erhebliche Abnahme des Umsatzes bezogen auf Reaktorvolumen und Zeit zur Folge.

Nach den bekannten Verfahren kann man also entweder Methanol mit zufriedenstellender Selektivität in höhere Alkohole überführen, erzielt dann aber nur geringe Umsätze oder man erreicht hohe Umsätze bei niedriger Selektivität.

Es bestand daher die Aufgabe, eine Arbeitsweise zu entwickeln, die es erlaubt, Methanol in Ethanol und n-Propanol mit hoher Selektivität und hohem Umsatz zu überführen, sowie die Anzahl der Nebenprodukte wesentlich zu reduzieren, so daß das Reaktionsgemisch in einfacher Weise aufgetrennt werden kann.

Die Erfindung löst diese Aufgabe mittels eines Verfahrens zur Herstellung von Ethanol und n-Propanol durch Umsetzung von Methanol mit Kohlenmonoxid und Wasserstoff bei erhöhten Drücken von 200 bis 600 bar und Temperaturen von 150 bis 250°C in Gegenwart eines Katalysators, der eine Kobaltverbindung, eine Rutheniumverbindung, ein Jodid und ein organisches Phosphin enthält. Es ist dadurch gekennzeichnet, daß das organische Phosphin eine Gruppe enthält, die einen Rest $-SO_3M$, $-OSO_3M$ oder $-COOM$ aufweist, wobei M für H, Alkalimetalle oder Ammonium steht. Nach einer besonders bevorzugten Ausführungsform beträgt das Volumenverhältnis $CO : H_2$ gleich 1 : 2,0 bis 1 : 3,5, das molare Verhältnis von Kobalt zu Methanol gleich 1 : 30 bis 1 : 5000, von Kobalt zu Jod gleich 1 : 0,1 bis 1 : 2, von Kobalt zu Ruthenium gleich 1 : 0,01 bis 1 : 0,05 und von Kobalt zu Phosphor gleich 1 : 0,5 bis 1 : 2,5.

Es war überraschend festzustellen, daß durch den Einsatz von derartigen Phosphinen eine erhebliche Verbesserung der Umsätze von Methanol und der Selektivitäten zu Ethanol und n-Propanol erreicht wird. Wie der Vergleich unter Verwendung von Triphenylphosphin, das keine $-SO_3M$, $-OSO_3M$ oder -COOM-Gruppe besitzt, in Vergleichsbeispiel 7 zeigt, werden erfindungsgemäß erheblich gesteigerte Umsätze und Selektivitäten unter gleichen Reaktionsbedingungen, wie in den Beispielen 1 bis 6 belegt, erreicht.

Als organische Phosphine, die im Rahmen der vorliegenden Erfindung eingesetzt werden können, haben sich besonders Verbindungen mit den allgemeinen Formeln

$$R_1R_2P\,[(CH\,R_3)_m-COOH] \qquad (I)$$

oder

$$R_1P\,[(CH\,R_2)_m-COOH] \qquad (II)$$

bewährt.

Hierbei steht m für die Zahlen von 1 bis 6 und die Reste $R_1$ bis $R_3$ sind gleich oder verschieden und stehen für Wasserstoff, geradkettige oder verzweigte (auch bicyclische) Alkylreste mit 1 bis 16 Kohlenstoffatomen oder Arylreste mit 6 bis 15 Kohlenstoffatomen.

Bei (I) können die Reste $R_1$ und $R_2$ paarweise miteinander verbunden sein und auf diese Weise ein Phosphor enthaltendes cyclisches oder bicyclisches System bilden.

Beispiele für Alkylreste sind Methyl, Ethyl, Propyl, Butyl, Pentyl, i-Propyl, i-Hexadecyl, Neopentyl, für Cycloalkylreste Cyclohexyl, Dicyclopentyl, Tricyclo-$(5,2,1,0^{2,6})$decenyl und für Arylreste Phenyl, Tolyl, Naphthyl, Phthalyl.

Beispiele für untereinander verbundene Alkylreste sind z. B.

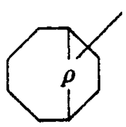

(9)-Phosphabicyclo (4,2,1)nonan

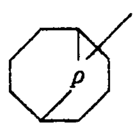

(9)-Phosphabicyclo (3,3,1)nonan

Beispiele für Verbindungen entsprechend den beschriebenen Formeln, die im Rahmen der erfindungsgemäßen Arbeitsweise eingesetzt werden, sind

Bis-(Tricyclo$(5,2,1,0^{2,6})$decenyl)-2-carboxyethylphosphin (1),
2-Carboxyethyl-dicyclohexylphosphin (2),
Bis-(2-carboxyethyl)-cyclohexyl-phosphin (3) und
Gemisch aus 9-(2-Carboxyethyl-9-phosphabicyclo(4,2,1)nonan und
9-(2-Carboxyethyl)-9-phosphabicyclo(3,3,1)nonan (4)

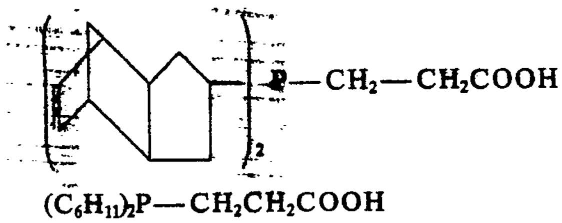

(1)

$(C_6H_{11})_2P—CH_2CH_2COOH$ (2)

$(C_6H_{11})P(CH_2CH_2COOH)_2$ (3)

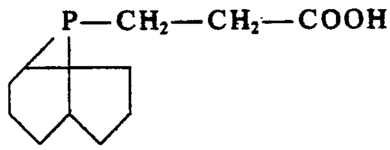

[3,3,1]

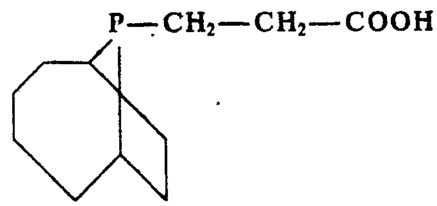

[4,2,1]

(4)

Weitere geeignete Phosphine, die im Rahmen der erfindungsgemäßen Arbeitsweise eingesetzt werden, sind das Trinatriumsalz von (2-Sulfatopropyl)-bis(m-sulfophenyl)-phosphin (5) und (Bis-1,3-(m-sulfophenyl)phenyl-phosphino)propan (6)

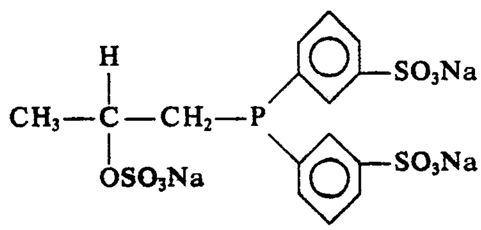

(5)

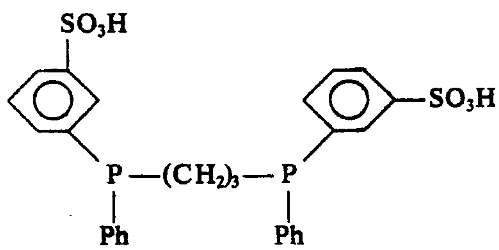

(6)

Die vorstehend beschriebenen Phosphorverbindungen mit (—COOM)-Gruppen sind durch radikalische Addition einer P—H-Bindung an eine C—C-Doppelbindung zugänglich (vgl. z. B. DE-OS 2 939 588 und J. Org. Chem., 26 [1961] 138). Die Darstellung der Verbindungen 1 bis 4 erfolgt durch Reaktion zwischen den entsprechenden mono- bzw. ditertiären Phosphinen und Acrylsäure. Im Falle von monotertiären Phosphinen werden zwei Acrylsäurereste addiert (vgl. Verb. 3).

Das Trinatriumsalz von (2-Sulfato-propyl)-bis(m-sulfophenyl)phosphin (5) und (Bis-1,3-(m-sulfophenyl)phenyl-phosphino)propan (6) sind durch Sulfonierung von Allyldiphenylphosphin bzw. 1,3-Diphenyl-phosphino-propan zugänglich.

Diese Phosphorverbindungen bilden mit den Kobalt- und Rutheniumverbindungen unter den Reaktionsbedingungen Komplexverbindungen, die außerdem Halogen, Kohlenmonoxid und Wasserstoff enthalten können. Diese Verbindungen stellen einen Bestandteil des wirksamen Katalysatorsystems dar.

Kobalt wird dem Reaktionsgemisch im allgemeinen in Form eines Salzes wie Kobalt-2-ethylhexanoat, Kobaltacetylacetonat, Kobalthalogenid, Kobaltnitrat, als Oxid oder Hydroxid zugesetzt. Besonders bewährt hat sich Kobaltcarbonat. Es ist aber auch möglich, metallisches Kobalt in feinverteilter Form zu verwenden.

Entscheidend ist, daß Kobalt bzw. die Kobaltverbindung mit Kohlenmonoxid und Wasserstoff unter Bildung eines Kobalt-Carbonyls oder -Hydrocarbonyls reagiert.

Ein weiterer Bestandteil des Katalysatorsystems entsprechend dem neuen Verfahren ist Ruthenium, das dem Reaktionsgemisch als Verbindung, die sich unter den Reaktionsbedingungen in Carbonyl komplexe umsetzt, z. B. Rutheniumhalogenid, Ruthenium-2-ethylhexanoat, Ruthenium chlorid, $(NH_4)_4(Ru_2OCl_{10})$, vorzugsweise als Rutheniumacetylacetonat zugesetzt wird.

Schließlich enthält das Katalysatorsystem noch Jod in ionischer Form. Als Salze des Jods können Alkalimetallsalze und besonders vorteilhaft Kobaltjodid verwendet werden.

Das im erfindungsgemäßen Verfahren eingesetzte Katalysatorsystem kann man dem Reaktionsgemisch in Form seiner einzelnen Bestandteile zuführen. Eine Präformierung der Metallkomplexverbindungen, die Komponenten des Katalysatorsystems sind, ist nicht erforderlich. Das Katalysatorsystem ist wiederholt einsetzbar.

Das als Ausgangsstoff eingesetzte Methanol kann in Form des in technischen Anlagen hergestellten Produktes mit einem Wassergehalt von 4 bis 6% verwendet werden. Eine zusätzliche Reinigung ist nicht erforderlich.

Das ursprünglich eingesetzte Reaktionsgemisch enthält 5 bis 25 Gew.-% Wasser, bezogen auf Methanol. Durch den Wasserzusatz wird eine Erhöhung des Umsatzes bewirkt. Höhere Wassermengen beeinflussen den Umsatz nur unwesentlich, geringere Mengen haben keine oder nur unerhebliche Umsatzsteigerungen zur Folge. Zweckmäßig wird das Wasser zusammen mit dem Methanol dem Reaktor zugeführt.

Das molare Verhältnis von Kobalt zu Methanol beträgt 1 : 30 bis 1 : 5000. Kobalt und Phosphin werden in einem molaren Verhältnis von 1 : 0,5 bis 1 : 2,5 eingesetzt.

Das Atomverhältnis von Kobalt zu Ruthenium beträgt 1 : 0,01 bis 1 : 0,05.

Kobalt und Jod werden im molaren Verhältnis von 1 : 0,1 bis 1 : 2 angewandt.

Das Kohlenmonoxid-/Wasserstoff-Gemisch soll keine die Aktivität des Katalysatorsystems beeinflussenden Verunreinigungen wie Schwefel enthalten. Kohlendioxid, inerte Kohlenwasserstoffe und/oder Stickstoff bis zu 5 Vol.-% bezogen auf das Gesamtgemisch schaden nicht.

Der neue Prozeß kann sowohl diskontinuierlich als auch kontinuierlich durchgeführt werden. Im allgemeinen erfolgt die Umsetzung von Methanol, Kohlenmonoxid und Wasserstoff bei Temperaturen von 150 bis 250°C insbesondere 160 bis 230°C. Der Druck wird auf Werte zwischen 200 bis 600 bar vorzugsweise 450 bis 600 bar gehalten. Das molare Verhältnis von Wasserstoff zu Kohlenmonoxid im Synthesegas beträgt 1 : 2,0 bis 1 : 3,5.

Die nachstehend beschriebenen Beispiele erläutern die Erfindung.

Die in den Tabellen angegebenen Selektivitäten wurden nach folgender Definition[1]) berechnet:

$$\text{Selektivität zu Produkt i: } (\%) = \frac{\text{Molzahl Produkt i} \times 100}{\text{Summe der Molzahlen aller erhaltenen Produkte}}.$$

## Beispiel 1

In einem Stahlautoklaven (1 l Inhalt), versehen mit Rührer, Temperaturmessung, Probenahmestutzen und einem Gasometer, das zum Auffangen gasförmiger Bestandteile dient, werden 9,375 mol (300 g) Methanol, 1,67 mol (30 g) Wasser, 25,22 mmol (3 g $CoCO_3$) Co, 10 mmol (1,5 g NaJ) Jodid und 30,56 mmol (11,2 g) Bis-(Tricyclo(5,2,1,$0^{2,6}$)decenyl)-2-carboxyethylphosphin) und 0,83 mmol (0,33 g Ru(III)acetylacetonat) Ru vorgelegt. Anschließend wird mit Synthesegas ein Druck von 550 bar eingestellt, auf 200°C erhitzt und die Reaktion 3 Stunden lang unter Nachpressen von Synthesegas fortgesetzt.

Nach Abkühlen des Reaktionsgemisches und Entspannen in den Gasometer wird eine Durchschnittsprobe gewonnen, deren gaschromatographische Zusammensetzung in der Tabelle angegeben ist.

## Beispiel 2—4

Diese Versuche werden analog Beispiel 1 durchgeführt, als phosphinhaltige Komponenten werden in

Beispiel 2: 27,74 mmol (7,2 g)
2-Carboxyethyldicyclohexylphosphin

Beispiel 3: 24,33 mmol (6,3 g)
Bis-(2-carboxyethyl)-cyclohexylphosphin

und in

Beispiel 4: 27,73 mmol (6,6 g) Gemisch aus
9-(2-Carboxyethyl)-9-phosphabicyclo(4,2,1)nonan und
9-(2-Carboxyethyl)-9-phosphabicyclo(3,3,1)nonan

eingesetzt. Die gaschromatographische Zusammensetzung der einzelnen Reaktionsprodukte ist in Tabelle 1 angegeben.

## Beispiel 5

In den Stahlautoklaven der Apparatur gemäß Beispiel 1 werden 9,375 mol (300 g) Methanol, 1,67 mol (30 g) Wasser, 12,61 mmol (1,5 g $CoCO_3$) Co, 12,68 mmol (1,9 g) Natriumjodid, 0,43 mmol (0,17 g $Ru(acac)_3$) Ru sowie 27,8 mmol (15,3 g) des Trinatriumsalzes von (2-Sulfato-propyl)bis-(m-sulfophenyl)phosphin vorgelegt, mit Synthesegas $CO/H_2 = 1:3$ ein Druck von 550 bar eingestellt, auf 200°C erhitzt und die Reaktion 3 Stunden lang unter Nachpressen von Synthesegas fortgesetzt. Nach Abkühlen des Gemisches und Entspannen in den Gasometer wird eine Durchschnittsprobe entnommen, deren gaschromatographische Zusammensetzung in Tabelle 1 angegeben ist.

## Beispiel 6

Der Versuch wird analog Beispiel 5 durchgeführt, als Phosphinkomponente werden 13,99 mmol (8,0 g) (Bis-1,3-(m-sulfophenyl)-phenyl-phosphino)propan eingesetzt.

## Vergleichsbeispiel 7

Unter den gleichen Versuchsbedingungen wie in Beispiel 5 werden 14,1 mmol (3,7 g) Triphenylphosphin als Phosphinkomponente eingesetzt.

Die Versuchsergebnisse aller beschriebenen Beispiele sind in Tabelle 2 aufgelistet.

Tabelle 1

GLC-Analyse (Gew.-%)

| | Versuchs-Nr. 1 | 2 | 3 | 4 | 5 | 6 | 7 |
|---|---|---|---|---|---|---|---|
| Methanol | 43,3 | 64,9 | 43,1 | 33,0 | 83,2 | 45,7 | 85,5 |
| Ethanol | 38,4 | 29,0 | 45,4 | 51,3 | 12,9 | 45,6 | 10,2 |
| n-Propanol | 1,5 | 1,2 | 3,8 | 4,9 | 0,2 | 2,0 | 0,1 |
| Kohlenwasserstoff | 2,9 | 1,6 | 0,3 | 1,3 | 0,4 | 1,7 | 0,2 |
| Acetaldehyd | — | — | 0,2 | 0,2 | — | — | 0,2 |
| Ether | 12,1 | 1,5 | 1,1 | 3,2 | 0,7 | 2,6 | 0,5 |
| Ester | 1,0 | 0,9 | 1,8 | 3,0 | 0,4 | 1,2 | 0,6 |
| Acetale | — | 0,4 | 1,5 | 1,0 | 1,8 | — | 1,9 |
| $C_4-C_8$-ole | 0,4 | 0,4 | 2,3 | 1,7 | 0,3 | 1,0 | 0,6 |
| Rest Nachlauf | 0,3 | 0,1 | 0,5 | 0,4 | 0,1 | 0,2 | 0,2 |

Tabelle 2

Umsätze und Selektivitäten

| | Beispiel 1 | 2 | 3 | 4 | 5 | 6 | 7 |
|---|---|---|---|---|---|---|---|
| Umsatz (%) | 56 | 39 | 62 | 62 | 15 | 59 | 15 |
| Selektivitäten % | | | | | | | |
| Ethanol | 69 | 78 | 81 | 80 | 78 | 80 | 62 |
| n-Propanol | 2 | 3 | 5 | 6 | 1 | 3 | — |
| Kohlenwasserstoff | 9 | 13 | 6 | 6 | 9 | 10 | 6 |
| Acetaldehyd | — | — | — | — | — | — | 1 |
| Ether | 17 | 3 | 2 | 4 | 4 | 4 | 5 |
| Ester | 2 | 2 | 2 | 2 | 2 | 1 | 3 |
| Acetale | — | — | 1 | 1 | 5 | — | 21 |
| Höhere Alkohole | 1 | 1 | 2 | 1 | 1 | 1 | 1 |
| Rest | — | — | 1 | — | — | 1 | 1 |

**Patentansprüche**

1. Verfahren zur Herstellung von Ethanol und n-Propanol durch Umsetzung von Methanol mit Kohlenmonoxid und Wasserstoff bei Drücken von 200 bis 600 bar und Temperaturen von 150 bis 250°C in Gegenwart eines Katalysators, der eine Kobaltverbindung, eine Rutheniumverbindung, ein Jodid und ein organisches Phosphin enthält, dadurch gekennzeichnet, daß das Phosphin eine Gruppe enthält, die einen Rest $-SO_3M$, $-COOM$ oder $-OSO_3M$ aufweist, wobei M für H, Alkalimetalle oder Ammonium steht.

2. Verfahren nach Anspruch 1, dadurch gekennzeichnet, daß das Volumenverhältnis CO : $H_2$ gleich 1 : 2,0 bis 1 : 3,5, das molare Verhältnis von Kobalt zu Methanol gleich 1 : 30 bis 1 : 5000, von Kobalt zu Jodid gleich 1 : 0,1 bis 1 : 2, von Kobalt zu Ruthenium gleich 1 : 0,01 bis 1 : 0,05 und von Kobalt zu Phosphor gleich 1 : 0,5 bis 1 : 2,5 beträgt.

3. Verfahren nach den Ansprüchen 1 und 2, dadurch gekennzeichnet, daß das Phosphin den allgemeinen Formeln

$$R_1R_2P[(CH\,R_3)_m-COOH]$$

oder

$$R_1P[(CH\,R_2)_m-COOH]_2$$

entspricht, wobei m für die Zahlen von 2 bis 6 steht und $R_1$, $R_2$ und $R_3$ gleich oder verschieden und für Wasserstoff, geradkettige oder verzweigte Alkylreste mit jeweils 1 bis 16 Kohlenstoffatomen oder Arylreste mit 6 bis 15 Kohlenstoffatomen stehen.

4. Verfahren nach den Ansprüchen 1 bis 3, dadurch gekennzeichnet, daß das Phosphin der allgemeinen Formel

$$R_1R_2P[(CH\,R_3)_m-COOH]$$

entspricht und der Phosphor und die Reste $R_1$ und $R_2$ ein gemeinsames cyclisches oder bicyclisches System bilden.

5. Verfahren nach den Ansprüchen 1 bis 3, dadurch gekennzeichnet, daß als Phosphinkomponente Bis-(Tricyclo(5,2,1,$0^{2,6}$)decenyl)-2-carboxyethylphosphin oder 2-Carboxyethyl-dicyclohexylphosphin oder Bis-(2-carboxyethyl)-cyclohexyl-phosphin eingesetzt wird.

6. Verfahren nach den Ansprüchen 1 bis 4, dadurch gekennzeichnet, daß ein Gemisch aus 9-(2-Carboxyethyl)-9-phosphabicyclo(4,2,1)nonan und 9-(2-Carboxyethyl)-9-phosphabicyclo(3,3,1) nonan eingesetzt wird.

7. Verfahren nach den Ansprüchen 1 und 2, dadurch gekennzeichnet, daß als Phosphin das Trinatriumsalz von (2-Sulfatopropyl)-bis(m-sulfophenyl)phosphin oder (Bis-1,3-(m-sulfophenyl)phenylphosphino)propan eingesetzt wird.

**Claims**

1. Process for producing ethanol and n-propanol by reacting methanol with carbon monoxide and hydrogen at pressures of 200 to 600 bars and temperatures of 150 to 250°C in the presence of a catalyst containing a cobalt compound, a ruthenium compound, an iodide and an organic phosphine, characterised in that the phosphine contains a group possessing a $-SO_3M$, $-COOM$ or $-OSO_3M$ radical, wherein M denotes H, alkali metals or ammonium.

2. Process according to claim 1, characterised in that the CO : $H_2$ volume ratio is 1 : 2.0 to 1 : 3.5, the molar ratio of cobalt to methanol is 1 : 30 to 1 : 5000, of cobalt to iodide is 1 : 0.1 to 1 : 2, of cobalt to ruthenium is 1 : 0.01 to 1 : 0,05, and of cobalt to phosphorus is 1 : 0.5 to 1 : 2.5.

3. Process according to claims 1 and 2, characterised in that the phosphine corresponds to the general formulae

$$R_1R_2P\,[(CH\,R_3)_m-COOH]$$

or

$$R_1P\,[(CH\,R_2)_m-COOH]_2$$

wherein m denotes the integers from 2 to 6 and $R_1$, $R_2$ and $R_3$ are the same or different and denote hydrogen, straightchain or branched alkyl radicals with in each case 1 to 16 carbon atoms, or aryl radicals with 6 to 15 carbon atoms.

4. Process according to claims 1 to 3, characterised in that the phosphine corresponds to the general formula

$$R_1R_2P\,[(CH\,R_3)_m-COOH]$$

and the phosphorus atom and the radicals $R_1$ and $R_2$ form a joint cyclic or bicyclic system.

5. Process according to claims 1 to 3, characterised in that bis-(tricyclo(5,2,1,0$^{2,6}$)decenyl)-2-carboxyethyl-phosphine or 2-carboxyethyl-dicyclohhexylphosphine or bis-(2-carboxyethyl)-cyclohexylphosphine is used as the phosphine component.

6. Process according to claims 1 to 4, characterised in that a mixture of 9-(2-carboxyethyl)-9-phosphabicyclo(4,2,1)nonane and 9-(2-carboxyethyl)-9-phosphabicyclo(3,3,1)nonane is used.

7. Process according to claims 1 and 2, characterised in that the trisodium salt of (2-sulphatopropyl)-bis(m-sulphophenyl)phosphine or (bis-1,3,-(m-sulphophenyl)phenylphosphino)propane is used as phosphine.

**Revendications**

1. Procédé de préparation de l'éthanol et du n-propanol par réaction du méthanol avec l'oxyde de carbone et l'hydrogène à des pressions de 200 à 600 bars et des températures de 150 à 250°C, en présence d'un catalyseur contenant un composé du cobalt, un composé du ruthénium, un iodure et une phosphine organique, caractérisé en ce que la phosphine contient un groupe portant un reste $-SO_3M$, $-COOM$ ou $-OSO_3M$, M représentant H, un métal alcalin ou l'ammonium.

2. Procédé selon la revendication 1, caractérisé en ce que le rapport en volume $COH_2$ va de 1 : 2,0 à 1 : 3,5, le rapport molaire cobalt/méthanol va de 1 : 30 à 1 : 5000, le rapport molaire cobalt/iodure va de 1 : 0,1 à 1 : 2, le rapport molaire cobalt/ruthénium va de 1 : 0,01 à 1 : 0,05 et le rapport molaire cobalt/phosphore va de 1 : 0,5 à 1 : 2,5.

3. Procédé selon ia revendications 1 et 2, caractérisé en ce que la phosphine répond aux formules générales

$$R_1R_2P\,[(CH\,R_3)_m-COOH]$$

ou

$$R_1P\,[(CH\,R_2)_m-COOH]_2$$

m étant un nombre de 2 à 6 et $R_1$, $R_2$ et $R_3$, ayant des significations identiques ou différentes, représentent l'hydrogène, des groupes alkyle à chaîne droite ou ramifiée contenant chacun 1 à 16 atomes de carbone ou des groupes aryle contenant 6 à 15 atomes de carbone.

4. Procédé selon les revendications 1 à 3, caractérisé en ce que la phosphine répond à la formule générale

$$R_1R_2P[(CH\,R_3)_m-COOH]$$

et le phosphore et les restes $R_1$ et $R_2$ forment un système cyclique ou bicyclique commun.

5. Procédé selon les revendications 1 à 3, caractérisé en ce que l'on utilise en tant que composant phosphine la bis-(tricyclo(5,2,1,$0^{2,6}$)décényl)-2-carboxyéthyl-phosphine ou la 2-carboxyéthyl-dicyclohexyl-phosphine ou la bis-(2-carboxyéthyl)-cyclohexyl-phosphine.

6. Procédé selon les revendication 1 à 4, caractérisé en ce que l'on utilise un mélange de 9-(2-carboxyéthyl)-9-phosphabicyclo(4,2,1)nonane et de 9-(2-carboxyéthyl)-9-phosphabicyclo(3,3,1)nonane.

7. Procédé selon les revendication 1 et 2, caractérisé en ce que l'on utilise en tant que phosphine le sel trisodique de la (2-sulfatopropyl)-bis-(m-sulfophényl)-phosphine ou le (bis-1,3-(m-sulfopényl)-phénylphosphino)-propane.